(19) Europäisches Patentamt
European Patent Office
Office européen des brevets

(11) Veröffentlichungsnummer: **0 135 762**
**B1**

(12) # EUROPÄISCHE PATENTSCHRIFT

(45) Veröffentlichungstag der Patentschrift: **12.10.88**

(51) Int. Cl.⁴: **C 07 J 51/00**, A 61 K 31/685

(21) Anmeldenummer: **84109517.7**

(22) Anmeldetag: **09.08.84**

(54) **Steroide.**

(30) Priorität: **25.08.83 CH 4644/83**

(43) Veröffentlichungstag der Anmeldung: **03.04.85 Patentblatt 85/14**

(45) Bekanntmachung des Hinweises auf die Patenterteilung: **12.10.88 Patentblatt 88/41**

(84) Benannte Vertragsstaaten: **AT BE CH DE FR GB IT LI LU NL SE**

(56) Entgegenhaltungen:
**THE JOURNAL OF ORGANIC CHEMISTRY, Band 43, Nr. 12, 9. Juni 1978, Seiten 2331-2334, Washington, DC, US; F. RAMIREZ u.a.: "Synthesis of lecithin analogues by means of cyclic enediol phosphates. Derivatives of 1-octadecanol and of cholesterol"**
**SYNTHESIS, no. 10, Oktober 1977, Seiten 673-674, Thieme, Stuttgart, DE; F. RAMIREZ u.a.: "Synthesis of phospholiposteroids. Phosphatidycholesterol"**

(73) Patentinhaber: **F. HOFFMANN- LA ROCHE & CO. Aktiengesellschaft, CH- 4002 Basel (CH)**

(72) Erfinder: **Cassal, Jean- Marie, Dr., 44 rue du Markstein, F-68100 Mulhouse (FR)**

(74) Vertreter: **Lederer, Franz, Dr., Van der Werth, Lederer & Riederer Patentanwälte Lucile- Grahn-Strasse 22, D-8000 München 80 (DE)**

**EP 0 135 762 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung betrifft neue Steroide der Formel

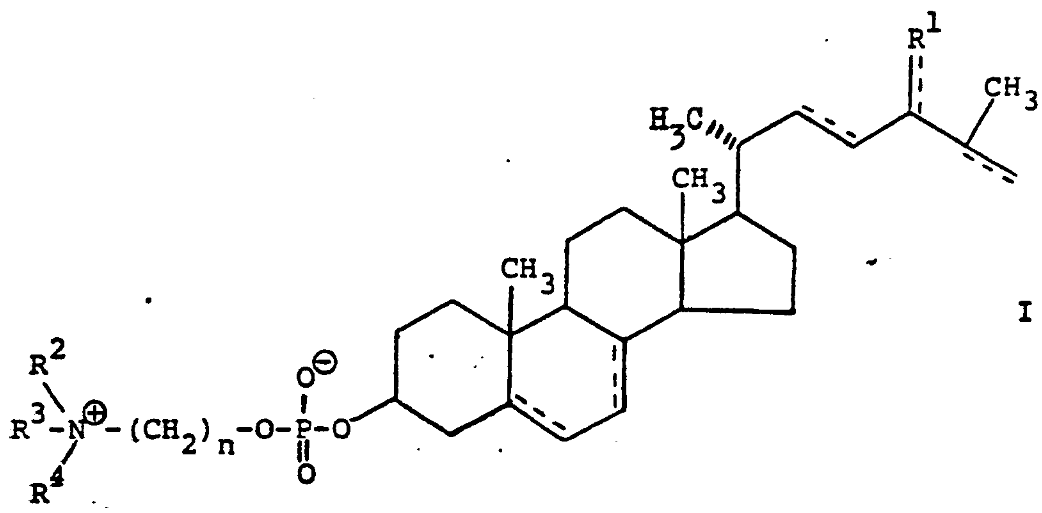

I

worin n die Zahl 2, 3 oder 4; $R^1$ Wasserstoff, nieder-Alkyl oder nieder-Alkyliden; $R^2$, $R^3$ und $R^4$ Wasserstoff oder nieder-Alkyl darstellen und die gestrichelten C-C-Bindungen in 5(6)-, 7(8)-, 22(23)-, 24(28)- und 25(26)-Stellung fakultativ sind, wobei der B-Ring nur eine Doppelbindung enthalten kann und die Seitenkette entweder gesättigt oder einfach ungesättigt oder in 22(23), 25(26)-Stellung zweifach ungesättigt ist; und wobei $R^1$ nieder-Alkyl oder nieder-Alkyliden ist, wenn eine 5(6)-Doppelbindung anwesend ist, n die Zahl 2 und $R^2$, $R^3$ und $R^4$ Methyl sind, und pharmazeutisch annehmbare Salze dieser Steroide.

Die erfindungsgemässen Steroide unterscheiden sich strukturell von dem in J. Org. Chem. 43 (1978) 2331 beschriebenen 3-Cholest-5-enylphosphorylcholin. Ferner zeichnen sie sich durch wertvolle pharmakologische Eigenschaften aus und können bei der Bekämpfung bzw. Verhütung von Krankheiten verwendet werden.

Die Erfindung betrifft weiterhin ein Verfahren zur Herstellung dieser Steroide, Arzneimittel auf der Basis dieser Steroide, und diese Steroide als pharmazeutische Wirkstoffe, insbesondere bei der Hemmung der intestinalen Cholesterinresorption.

Der Ausdruck "nieder" bedeutet, dass die derart bezeichneten Reste bis zu 4 Kohlenstoffatome enthalten und geradkettig oder verzweigt sein können. Beispiele von nieder-Alkylresten sind Methyl, Äthyl, n-Propyl, Isopropyl, n-Butyl und t-Butyl.

Pharmazeutisch annehmbare Salze der Steroide der Formel I sind anorganische Salze, wie Hydrochloride und Sulfate; organische Salze, wie Trifluoracetate, Mesylate und Tosylate; und Metallsalze, wie Natriumsalze. Die Verbindungen der Formel I, in denen mindestens einer der Reste $R^2$, $R^3$ und $R^4$ Wasserstoff ist, können in Form eines Zwitterions oder eines Hydrogenphosphats vorliegen.

Unter den Steroiden der Formel I sind diejenigen bevorzugt, worin $R^1$ Wasserstoff oder nieder-Alkyl, insbesondere Äthyl ist, ferner diejenigen, worin $R^2$, $R^3$ und $R^4$ nieder-Alkyl, insbesondere Methyl sind, sowie diejenigen, worin n die Zahl 2 ist. Bevorzugt sind auch die gesättigten Steroide der Formel I, sowie die 5(6)- und 22(23)-ungesättigten. Besonders bevorzugt ist das 3β-Stigmastanyloxyphosphorylcholin und das Stigmasta-5,22-dien-3β-yloxy-phosphorylcholin.

Die Steroide der Formel I und die Salze davon können dadurch hergestellt werden, dass man

a) eine Verbindung der Formel

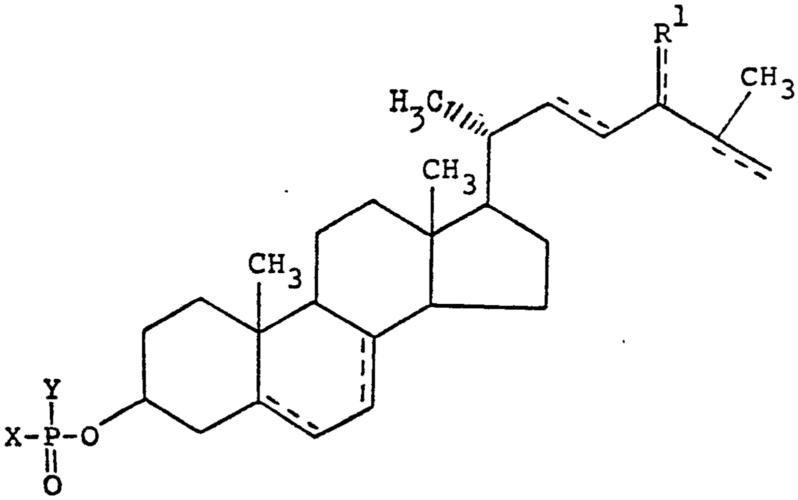

II

mit einem Salz der Formel

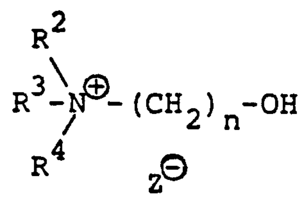

III

worin n, $R^1$, $R^2$, $R^3$, $R^4$ und die gestrichelten C-C-Bindungen die oben angegebenen Bedeutungen haben; X und Y Chlor, Brom oder Jod, oder X zusammen mit Y den 1,2-Dimethyläthylendioxyrest, und Z nieder-Alkylsulfonyloxy, Arylsulfonyloxy, Perchloryloxy, Chloro, Bromo oder Jodo sind,

in Gegenwart einer Base umsetzt und das Reaktionsprodukt hydrolysiert, oder

b) eine Verbindung der Formel II, worin X eine metallierte Hydroxygruppe und Y eine Gruppe der Formel Hal-$(CH_2)_n$-O- ist, worin Hal Chlor, Brom oder Jod ist und n die obige Bedeutung hat, oder worin X zusammen mit Y den Äthylendioxyrest bildet, mit einem Amin der Formel $N(R^1,R^3,R^4)$, worin $R^2$, $R^3$ und $R^4$ die obige Bedeutung haben, umsetzt,

oder

c) in einer Verbindung der Formel II, worin X eine metallierte Hydroxygruppe und Y ein Rest der Formel

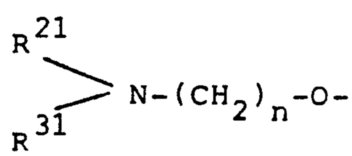

(Y)

ist,

worin eines von $R^{21}$ und $R^{31}$ eine Aminoschutzgruppe ist und das andere die Bedeutung von $R^2$ hat, oder $R^{21}$ und $R^{31}$ zusammen mit dem N-Atom eine geschützte Aminogruppe sind, und n und $R^2$ die obige Bedeutung haben,

die Aminoschutzgruppe abspaltet, und

d) ein erhaltenes Steroid der Formel I in dieser Form oder in Form eines Salzes isoliert.

Für die Herstellung der erfindungsgemässen Produkte geht man zweckmässigerweise so vor, dass man das Ausgangsmaterial der Formel II kurz oder unmittelbar vor der Umsetzung mit einer Verbindung der Formel III oder einem Amin der Formel $N(R^2,R^3,R^4)$ oder vor der Abspaltung der Aminoschutzgruppe auf die weiter unten beschriebene Weise aus einem entsprechenden Steroid der Formel IV herstellt und als Rohprodukt in die Reaktion einbringt.

In der Verfahrensvariante a) werden vorzugsweise Steroide der Formel II, worin X und Y Chlor sind, mit Salzen der Formel III, worin Z nieder-Alkyl- oder Arylsulfonyloxy ist, insbesondere mit Mesylaten oder Tosylaten, umgesetzt. Als Basen können organische Basen, wie Tri-(nieder-alkyl)-amine, z. B. Trimethyl- oder Triäthylamin; Chinolin oder Pyridin; oder anorganische Basen, wie Alkalimetall- oder Erdalkalimetallhydroxyde, -carbonate oder -bicarbonate wie $Na_2CO_3$, $KHCO_3$ oder $CaCO_3$, verwendet werden. Die Reaktion kann in einem

Lösungsmittel, wie einem halogenierten Kohlenwasserstoff z. B. Chloroform, Methylenchlorid oder Dichloräthylen; oder Acetonitril oder in einem Gemisch davon, durchgeführt werden.

Die darauf folgende Hydrolyse kann mit Wasser oder mit einer verdünnten Säure wie Salzsäure oder einer Base, wie einer der weiter oben erwähnten organischen oder anorganischen Basen durchgeführt werden.

In der Verfahrensvariante a) ist die Temperatur nicht kritisch. Vorzugsweise wird jedoch bei Raumtemperatur gearbeitet.

In der Verfahrensvariante b) werden zweckmässigerweise Steroide der Formel II eingesetzt, worin X eine mit einem Alkali- oder Erdalkalimetall metallierte Hydroxygruppe, vorzugsweise eine Gruppe -ONa und Y eine Gruppe der Formel Hal-$(CH_2)_n$-O-, vorzugsweise der Formel Cl-$(CH_2)_n$-O-, ist. Die Umsetzung eines solchen Steroids II mit einem Amin $N(R^2,R^3,R^4)$ wird zweckmässig in einem Lösungsmittel, wie einem halogenierten Kohlenwasserstoff, z. B. Chloroform, einem Alkohol, z. B. Äthanol oder 2-Propanol; in Acetonitril oder DMF, oder in einem Gemisch davon, durchgeführt. Dabei ist die Temperatur nicht kritisch. Vorzugsweise wird jedoch bei Raumtemperatur gearbeitet.

In der Verfahrensvariante b) kann auch ein Steroid der Formel II, worin X zusammen mit Y eine Äthylendioxygruppe bildet, mit einem Amin $N(R^2,R^3,R^4)$ umgesetzt werden. Die Umsetzung wird zweckmässigerweise unter Druck und unter Erwärmen, vorzugsweise unter 1 bis 20 Atm bei einer Temperatur zwischen 20 und 100°C, durchgeführt, wobei eines der für die Verfahrensvariante a) zitierten Lösungsmittel verwendet werden kann.

In der Verfahrensvariante c) wird zweckmässigerweise ein Steroid der Formel II umgesetzt, worin X eine mit einem Alkalimetall, vorzugsweise Natrium, metallierte Hydroxygruppe ist. Beispiele von im Rest Y enthaltenen Aminoschutzgruppen $R^{21}$ oder $R^{31}$ sind Benzyloxycarbonyl, Phenoxycarbonyl, Formyl, Trifluormethylcarbonyl, Trimethylsilyl und Trityl. Beispiele von geschützten Aminogruppen -$N(R^{21},R^{31})$ sind Succinimid und Phthalimidgruppen. Solche Aminoschutzgruppen kann man in an sich bekannter Weise abspalten, z. B. eine Benzyloxycarbonylgruppe durch Hydrogenolyse oder mittels Bromwasserstoffsäure und Essigsäure, die Tritylgruppe mittels Bromwasserstoffsaure und Essigsäure, und die Phthalimidgruppe mit Hydrazinhydrat in einem Alkohol, wie Methanol.

Die als Ausgangsstoffe verwendeten Steroide der Formel II kann man, wie bereits weiter oben erwähnt, aus entsprechenden Steroiden der Formel

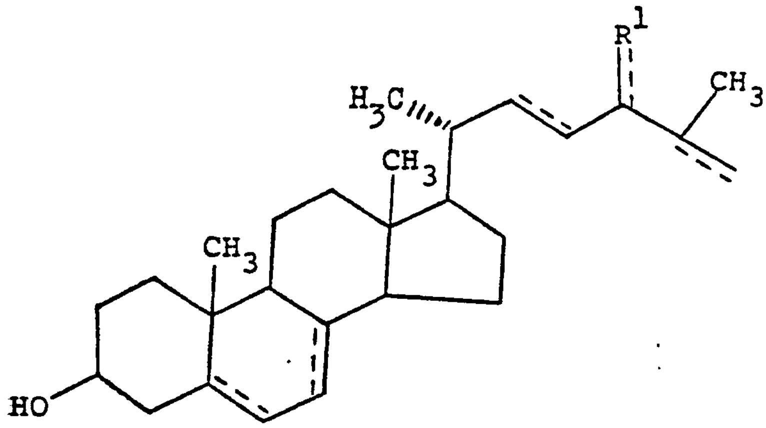

IV

worin $R^1$ und die gestrichelten C-C-Bindungen die obige Bedeutung haben, herstellen.

Die in der Verfahrensvariante a) eingesetzten Steroide der Formel II können dadurch hergestellt werden, dass man ein Steroid der Formel IV mit einer Verbindung der Formel $PO(Hal)_3$ oder $(Hal)_2POOPO(Hal)_2$ oder

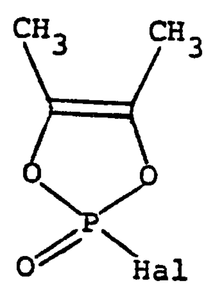

V

worin Hal Chlor, Brom oder Jod ist,

in Gegenwart einer Base, gegebenenfalls in einem Lösungsmittel umsetzt. Zweckmässigerweise wird eine Verbindung der Formel $PO(Hal)_3$, vorzugsweise $POCl_3$, oder 1,2-Dimethyläthenylendioxy-phosphorylchlorid

eingesetzt. Als Base und Lösungsmittel können die gleichen wie für die Verfahrensvariante a) verwendet werden.

Die in der Verfahrensvariante b) eingesetzten Steroide der Formel II, worin X eine metallierte Hydroxygruppe und Y eine Gruppe der Formel Hal-$(CH_2)_n$-O- ist, können dadurch hergestellt werden, dass man ein Steroid der Formel IV mit einer Verbindung der Formel

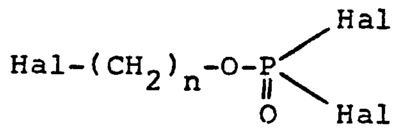

VI

worin Hal und n obige Bedeutung haben,

in Gegenwart einer Base in einem Lösungsmittel umsetzt und das Reaktionsprodukt mit einer verdünnten anorganischen Base in einem Lösungsmittel hydrolysiert. Als Base und Lösungsmittel für die Umsetzung der Verbindungen der Formeln IV und VI bzw. als anorganische Base für die anschliessende Hydrolyse können die gleichen wie für die Verfahrensvariante a) verwendet werden. Bei der Hydrolyse wird als Lösungsmittel ein Äther, wie THF oder Dioxan, oder ein Keton, wie Aceton, verwendet.

Die in der Verfahrensvariante b) eingesetzten Steroide der Formel II, worin X zusammen mit Y einen Äthylendioxyrest bildet, können dadurch hergestellt werden, dass man ein Steroid der Formel IV mit einer Verbindung der Formel

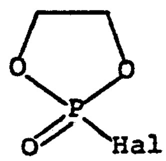

VII

worin Hal obige Bedeutung hat,

z. B. mit 2-Chlor-2-oxo-1,3,2-dioxaphospholan, in Gegenwart einer Base, gegebenenfalls in einem Lösungsmittel umsetzt. Als Base und Lösungsmittel kann man die gleichen wie für die Verfahrensvariante a) verwenden.

Die in der Verfahrensvariante c) eingesetzten Steroide der Formel II können dadurch hergestellt werden dass man ein Steroid der Formel IV mit einer Verbindung der Formel

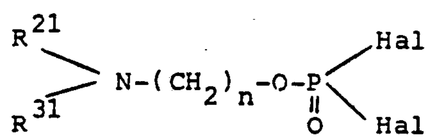

VIII

worin $R^{21}$, $R^{31}$, Hal und n obige Bedeutung haben, in Gegenwart einer Base, gegebenenfalls in einem Lösungsmittel, umsetzt. Als Base und Lösungsmittel kann man die gleichen wie für die Verfahrensvariante a) verwenden.

Die Steroide der Formel I hemmen die intestinale Resorption von Cholesterin.

Die Hemmung der intestinalen Resorption von Cholesterin lässt sich tierexperimentell wie folgt nachweisen:

Totenkopfäffchen werden die zu untersuchenden Substanzen zusammen mit einem Protein, Stärke, Triolein und [26-$^{14}$C]-Cholesterin enthaltenden Futter oral verabreicht. Hierauf wird der Kot während 2,5 Tagen gesammelt. Die im Kot ermittelte Differenz zwischen dem verabreichten und dem ausgeschiedenen radioaktiven Cholesterin wird als Mass für resorbiertes Cholesterin genommen. Die Cholesterinresorption (CHORES) wird in Prozenten der vor der Medikation ermittelten Kontrollwerte ausgedrückt.

In der nachstehenden Tabelle werden die Resultate wiedergegeben, welche mit einigen repräsentativen erfindungsgemässen Produkten erhalten worden sind. Angegeben werden für jede der darin figurierenden Verbindungen die verabreichte Dosis (in µMol/kg p.o.) sowie die jeweils ermittelte Cholesterinresorption (CHORES) in Prozenten der Cholesterinresorption in der Vorperiode. Ausserdem enthält die Tabelle Angaben über die akute Toxizität der untersuchten Verbindungen ($DL_{50}$ in mg/kg bei einmaliger oraler Verabreichung an Mäusen).

**Tabelle**

| Verbindung der Formel I | Dosis in µMol/kg p.o. | CHORES in % der Vorperiode | $DL_{50}$ in mg/kg p.o. |
|---|---|---|---|
| 3β-Cholestanyloxy-phosphorylcholin | 100 | 26 | 5000 |
| Stigmast-5-en-3ß-yloxy-phosphorylcholin | 100 | 52 | 5000 |
| Stigmasta-5,22-dien-3β-yloxy-phosphorylcholin | 100 | 33 | 5000 |
| 3β-Stigmastanyloxy-phosphorylcholin | 100 | 36 | 4000 |

Die erfindungsgemässen Produkte können als Heilmittel, z. B. in Form pharmazeutischer Präparate, Verwendung finden. Die pharmazeutischen Präparate werden oral, z. B. in Form von Tabletten, Lacktabletten, Dragées, Hart- und Weichgelatinekapseln, Lösungen, Emulsionen oder Suspensionen, verabreicht.

Zur Herstellung von pharmazeutischen Präparaten können die erfindungsgemässen Produkte mit pharmazeutisch inerten, anorganischen oder organischen Trägern verarbeitet werden. Als solche Träger kann man für Tabletten, Lacktabletten, Dragées und Hartgelatinekapseln beispielsweise Lactose, Maisstärke oder Derivate davon, Talk, Stearinsäure oder deren Salze und dergleichen verwenden. Für Weichgelatinekapseln eignen sich als Träger beispielsweise pflanzliche Öle, Wachse, Fette, halbfeste und flüssige Polyole und dergleichen; je nach Beschaffenheit des Wirkstoffes sind jedoch bei Weichgelatinekapseln überhaupt keine Träger erforderlich. Zur Herstellung von Lösungen und Sirupen eignen sich als Träger beispielsweise Wasser, Polyole, Saccharose, Invertzucker, Glukose und dergleichen.

Die pharmazeutischen Präparate können daneben noch Konservierungsmittel, Lösungsvermittler, Stabilisierungsmittel, Netzmittel, Emulgiermittel, Süssmittel, Färbemittel, Aromatisierungsmittel, Salze zur Veränderung des osmotischen Druckes, Puffer, Überzugsmittel oder Antioxidantien enthalten. Sie können auch noch andere therapeutisch wertvolle Stoffe enthalten.

Wie eingangs erwähnt sind Arzneimittel, enthaltend ein Steroid der Formel I oder ein pharmazeutisch annehmbares Salz davon ebenfalls Gegenstand der vorliegenden Erfindung, weiterhin auch ein Verfahren zur Herstellung solcher Arzneimittel, welches dadurch gekennzeichnet ist, dass man eine oder mehrere erfindungsgemässe Produkte und gegebenenfalls einen oder mehrere andere therapeutisch wertvolle Stoffe in eine galenische Darreichungsform bringt. Wie eingangs erwähnt können die erfindungsgemässen Produkte bei der Bekämpfung bzw. Verhütung von Krankheiten verwendet werden.

Sie können insbesondere bei der Bekämpfung bzw. Verhütung der Hypercholesterinämie und der Atherosklerose verwendet werden. Die Dosierung kann innerhalb weiter Grenzen variieren und ist natürlich in jedem einzelnen Fall den individuellen Gegebenheiten anzupassen. Im Allgemeinen dürfte bei oraler Verabreichung eine Tagesdosis von etwa 50 mg bis etwa 3 g, vorzugsweise von etwa 200 mg bis etwa 1 g angemessen sein.

Die nachfolgenden Beispiele sollen die vorliegende Erfindung näher erläutern, ihren Umfang jedoch in keiner Weise beschränken. Alle Temperaturen sind in Celsiusgraden angegeben.

**Beispiel 1**

Zu einer Lösung von 1,95 ml (20,8 mMol) Phosphoroxychlorid tropft man bei Raumtemperatur eine Lösung von 7,78 g (16,7 mMol) β-Sitosterol und 2,5 ml (20,8 mMol) Chinolin in 100 ml Chloroform. Die Lösung wird dann auf 45° erhitzt und dann mit 10 g (36,3 mMol) Cholintosylat und 10 ml Pyridin bei Raumtemperatur versetzt, worauf man das Reaktionsgemisch bei Raumtemperatur rührt. Man versetzt mit 3 ml Wasser. Das Gemisch wird in 150 ml Chloroform gelöst und nacheinander mit je 2 x 50 ml Wasser, 3 % Natriumcarbonatlösung, Wasser, 5 % HCl-Lösung und Wasser gewaschen. Die organische Phase wird getrocknet und eingedampft. Der Rückstand wird unter Eluieren mit Chloroform-Methanol-Wasser (40 : 55 : 5) an Kieselgel chromatographiert. Das Produkt wird durch azeotrope Destillation mit Toluol behandelt und dann in Chloroform-Äther umkristallisiert. Man erhält das Stigmast-5-en-3β-yloxy-phosphorylcholin vom Schmelzpunkt 285 - 286°.

**Beispiel 2**

In zu Beispiel 1 analoger Weise wird das Stigmasta-5,22-dien-3β-yloxy-phosphorylcholin vom Schmelzpunkt 228° (Zersetzung) hergestellt.

**Beispiel 3**

In zu Beispiel 1 analoger Weise wird das 3β-Cholestanyloxy-phosphorylcholin vom Schmelzpunkt 281 - 283° hergestellt.

**Beispiel 4**

<u>A. Herstellung des Ausgangsmaterials:</u>

a) Eine Lösung von 17 g (70,4 mMol) 2-Bromäthylphosphor-säuredichlorid in 90 ml Trichloräthylen wird auf 0 - 5° gekühlt und mit 14,26 g (141 mMol) Triäthylamin versetzt. Unter Argon tropft man unter Rühren bei 25° eine

Lösung von 16,28 g (39,07 mMol) Stigmastanol in 100 ml Trichloräthylen hinzu, worauf man die Temperatur der Lösung zwischen 25 - 30° hält. Die Lösung wird dann bei 25° gerührt und mit 180 ml Toluol versetzt. Das ausgefallene Triäthylamin-chlorhydrat wird abgenutscht. Das Filtrat wird unter vermindertem Druck bei 50° getrocknet. Man erhält das (2-Bromäthyl)-(3β-stigmastanyl)-phosphorchloridat in Form eines öligen Rückstands.

b) Dieser Rückstand wird in 160 ml Tetrahydrofuran gelöst und mit 350 ml 0,5M Natriumacetat versetzt. Die erhaltene Lösung (pH 4,5) wird gerührt. Man gibt 17,6 ml 0,5M E.D.T.A. zu und bringt den pH der Lösung auf 9,5 durch Zugabe von 45 ml 3N NaOH. Man extrahiert das Reaktionsgemisch mit 350 ml Diisopropyläther und 140 ml MeOH. Die organische Phase wird eingedampft. Der Rückstand wird aus Chloroform-Methanol-Aceton umkristallisiert, die erhaltenen Kristalle werden mit Aceton und Äther gewaschen und bei 45° getrocknet. Man erhält das Natrium-(2-bromäthyl)-(3β-stigmastanyl)-phosphat in Form von weissen Kristallen.

Zur Analyse werden 500 mg dieser Kristalle mit 2N HCl angesäuert und mit Äther extrahiert. Der Rückstand wird in Äther-n-Hexan umkristallisiert. Man erhält das (2-Bromäthyl)-(3β-stigmastanyl)-hydrogenphosphat vom Schmelzpunkt 129 - 130°.

<u>B. Herstellung des Produktes:</u>

Eine Lösung von 5 g (8 mMol) Natrium-(2-bromäthyl)-(3β-stigmastanyl)-phosphat in 25 ml Chloroform, 40 ml 2-Propanol und 40 ml Acetonitril wird mit 60 ml 45 % wässrigem Trimethylamin versetzt, bei Raumtemperatur gerührt und dann verdampft. Der Rückstand wird in 125 ml Wasser aufgenommen und mit 250 ml Chloroform-Methanol (1 : 1) extrahiert. Die organische Phase wird getrocknet und eingedampft. Durch Chromatographie an Kieselgel unter Eluieren mit $CHCl_3$-MeOH (8 : 2 bis 6 : 4) und dann mit $CHCl_3$-MeOH-$H_2O$ (60 : 35 : 5) und Umkristallisieren aus Chloroform-Äther erhält man das 3β-Stigmastanyloxy-phosphorylcholin vom Schmelzpunkt 270° (Zersetzung).

**Beispiel 5**

In zu Beispiel 4B analoger Weise erhält man unter Verwendung von Dimethylamin anstatt Trimethylamin das (2-Dimethylaminoäthyl)-(3β-stigmastanyl)-hydrogenphosphat vom Schmelzpunkt 267° (Zersetzung).

**Beispiel 6**

In zu Beispiel 4B analoger Weise erhält man unter Verwendung von Methylamin anstatt Trimethylamin das (2-Methylaminoäthyl)-(3β-stigmastanyl)-hydrogenphosphat vom Schmelzpunkt 258 - 260° (Zersetzung).

**Beispiel 7**

Eine Lösung von 5 g (8 mMol) Natrium-(2-bromäthyl)-(3β-stigmastanyl)-phosphat in 80 ml Chloroform, 80 ml 2-Propanol und 320 ml Dimethylformamid wird mit 240 ml 25 % Ammoniak versetzt, bei Raumtemperatur gerührt und dann eingeengt. Die wässrige DMF-Phase wird mit 2N Salzsäure angesäuert und mit 1,5 l $CHCl_3$-MeOH (50 : 50) extrahiert. Die organische Phase wird getrocknet und eingedampft. Durch Chromatographieren an Kieselgel unter Eluieren mit Chloroform-Methanol (7 : 3) und Chloroform-Methanol-Wasser (60 : 35 : 5) erhält man das (2-Aminoäthyl)-(3β-stigmastanyl)-phosphat-hydrochlorid vom Schmelzpunkt 202°.

**Beispiel 8**

In zu Beispiel 4 analoger Weise erhält man ausgehend von Stigmastanol und 3-Brompropylphosphorsäuredichlorid über das (3-Brompropyl)-(3β-stigmastanyl)-phosphorochloridat und das Natrium-(3-brompropyl)-(3β-stigmastanyl)-phosphat das 3-(3β-Stigmastanyloxy-phosphoryl)-propylamin vom Schmelzpunkt 270° (Zersetzung).

**Beispiel A**

3β-Stigmastanyloxy-phosphorylcholin kann wie folgt als Wirkstoff zur Herstellung von pharmazeutischen Präparaten verwendet werden:

a)

| Tabletten | 1 Tablette enthält |
|---|---|
| Wirkstoff | 200 mg |
| mikrokristalline Cellulose | 155 mg |
| Maisstärke | 25 mg |
| Talk | 25 mg |
| Hydroxypropylmethylcellulose | 20 mg |
| | 425 mg |

Der Wirkstoff wird mit der Hälfte der mikrokristallinen Cellulose gemischt und mit einer 10-proz. Lösung von Hydroxypropylmethylcellulose in einem Gemisch aus Isopropanol und Methylenchlorid granuliert. Das Granulat wird getrocknet, gesiebt und mit dem Rest der Hilfsstoffe gemischt. Alsdann wird es auf einer Presse zu biplanen Tabletten von 12 mm Durchmesser mit Kreuzbruchrille verpresst.

b)

| Kapseln | 1 Kapsel enthält |
|---|---|
| Wirkstoff | 100,0 mg |
| Maisstärke | 20,0 mg |
| Milchzucker | 95,0 mg |
| Talk | 4,5 mg |
| Magnesiumstearat | 0,5 mg |
| | 220,0 mg |

Der Wirkstoff wird mit den Hilfsstoffen gemischt und gesiebt. Nach erneutem Mischen wird die erhaltene Kapselfüllmasse auf einer vollautomatischen Kapselabfüllmaschine in Gelatinesteckkapseln geeigneter Grösse abgefüllt.

**Patentansprüche** für die Vertragsstaaten: BE, CH, DE, FR, GB, IT, LI, LU, NL, SE

1. Steroide der Formel

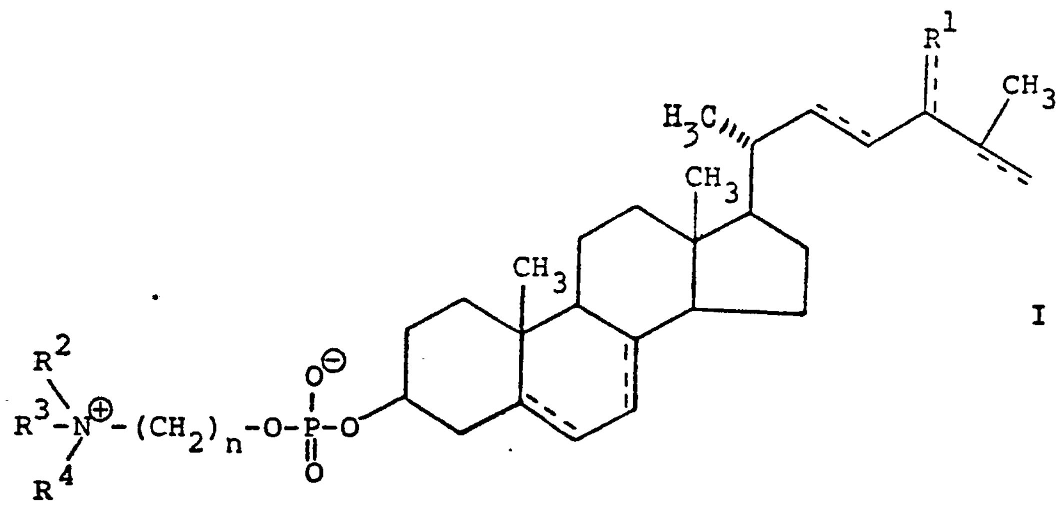

I

worin n die Zahl 2, 3 oder 4; $R^1$ Wasserstoff, nieder-Alkyl ($C_1$-$C_4$) oder nieder-Alkyliden ($C_2$-$C_4$); $R^2$, $R^3$ und $R^4$ Wasserstoff oder nieder-Alkyl ($C_1$-$C_4$) darstellen und die gestrichelten C-C-Bindungen in 5(6)-, 7(8)-, 22(23)-, 24(28)- und 25(26)-Stellung fakultativ sind, wobei der B-Ring nur eine Doppelbindung enthalten kann und die Seitenkette entweder gesättigt oder einfach ungesättigt oder in 22(23), 25(26)-Stellung zweifach ungesättigt ist; und wobei $R^1$ nieder-Alkyl ($C_1$-$C_4$) oder nieder-Alkyliden ($C_2$-$C_4$) ist, wenn eine 5(6)-Doppelbindung anwesend ist, n 2 und $R^2$, $R^3$ und $R^4$ Methyl sind,
und pharmazeutisch annehmbare Salze dieser Steroide.

2. Steroide nach Anspruch 1, worin $R^1$ Wasserstoff oder nieder-Alkyl ($C_1$-$C_4$), insbesondere Äthyl, ist.

3. Steroide nach Anspruch 1 oder 2, worin $R^2$, $R^3$ und $R^4$ nieder-Alkyl ($C_1$-$C_4$), insbesondere Methyl, sind.

4. Steroide nach Anspruch 1, 2 oder 3, worin n die Zahl 2 ist.

5. Gesättigte Steroide nach einem der Ansprüche 1 - 4.

6. 5(6)- und 22(23)-ungesättigte Steroide nach einem der Ansprüche 1 - 4.

7. 3β-Stigmastanyloxy-phosphorylcholin.

8. Stigmasta-5,22-dien-3β-yloxy-phosphorylcholin.

9. Produkte nach einem der Ansprüche 1 - 8 als pharmazeutische Wirkstoffe.

10. Produkte nach einem der Ansprüche 1 - 8 als die intestinale Resorption von Cholesterin hemmende Wirkstoffe.

11. Verfahren zur Herstellung von Produkten nach einem der Ansprüche 1 - 8, dadurch gekennzeichnet, dass man

a) eine Verbindung der Formel

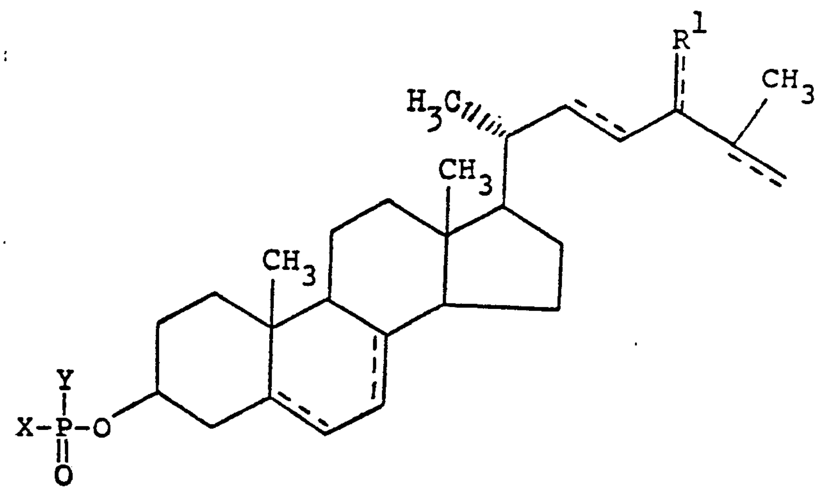

II

mit einem Salze der Formel

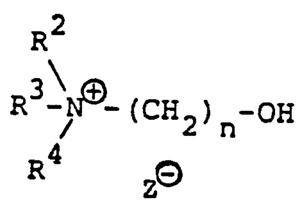

III

worin n, $R^1$, $R^2$, $R^3$, $R^4$ und die gestrichelten C-C-Bindungen die im Anspruch 1 angegebenen Bedeutungen haben; X und Y Chlor, Brom oder Jod, oder X zusammen mit Y einen 1,2-Dimethyläthylendioxyrest, und Z nieder-Alkylsulfonyloxy, Arylsulfonyloxy, Perchloryloxy, Chlor, Brom oder Jod sind,

in Gegenwart einer Base umsetzt und das Produkt hydrolysiert, oder

b) eine Verbindung der Formel II, worin X eine metallierte Hydroxygruppe und Y eine Gruppe der Formel Hal-$(CH_2)_n$-O-ist, worin Hal Chlor, Brom oder Jod ist; und n die obige Bedeutung hat, oder worin X zusammen mit Y einen Äthylendioxyrest bildet, mit einem Amin der Formel N($R^2$, $R^3$, $R^4$), worin $R^2$, $R^3$ und $R^4$ die obige Bedeutung haben, umsetzt, oder

c) in einer Verbindung der Formel II, worin X eine metallierte Hydroxygruppe und Y ein Rest der Formel

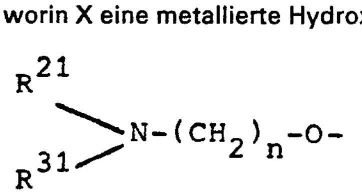

(Y)

ist,

worin eines von $R^{21}$ und $R^{31}$ eine Aminoschutzgruppe ist und das andere die Bedeutung von $R^2$ hat, oder $R^{21}$ und $R^{31}$ zusammen mit dem N-Atom eine geschützte Aminogruppe sind, und n und $R^2$ die obige Bedeutung haben,

die Aminoschutzgruppe abspaltet, und

d) ein erhaltenes Steroid der Formel I in dieser Form oder in Form eines Salzes isoliert.

12. Arzneimittel enthaltend eine Verbindung nach einem der Ansprüche 1 - 8.

13. Die intestinale Resorption von Cholesterin hemmende Mittel, enthaltend eine Verbindung nach einem der Ansprüche 1 - 8.

**Patentansprüche** für den Vertragsstaat: AT

1. Verfahren zur Herstellung von Steroiden der Formel

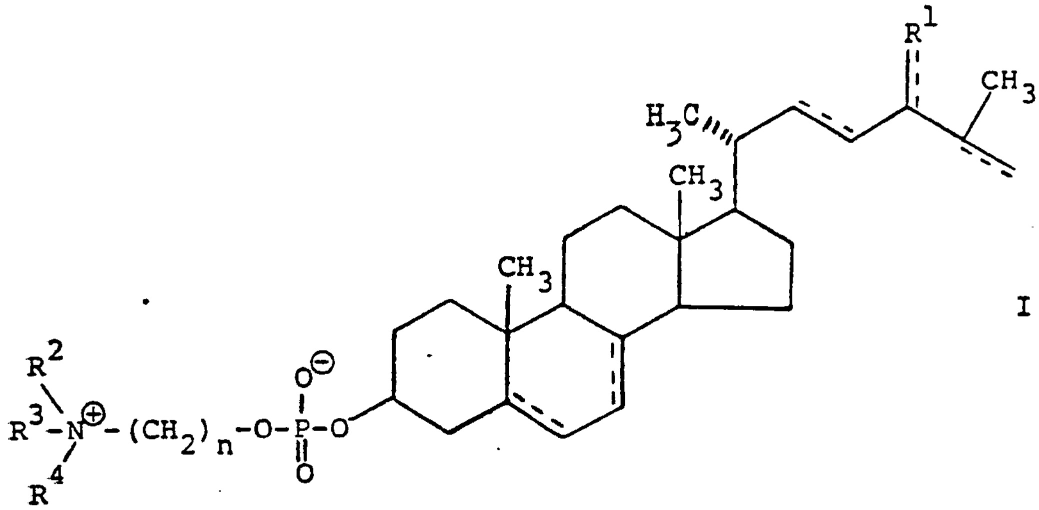

I

worin n die Zahl 2, 3 oder 4; $R^1$ Wasserstoff, nieder-Alkyl ($C_1$-$C_4$) oder nieder-Alkyliden ($C_2$-$C_4$); $R^2$, $R^3$ und $R^4$ Wasserstoff oder nieder-Alkyl darstellen und die gestrichelten C-C-Bindungen in 5(6)-, 7(8)-, 22(23)-, 24(28)- und 25(26)-Stellung fakultativ sind, wobei der B-Ring nur eine Doppelbindung enthalten kann und die Seitenkette entweder gesättigt oder einfach ungesättigt oder in 22(23), 25(26)-Stellung zweifach ungesättigt ist; und wobei $R^1$ nieder-Alkyl ($C_1$-$C_4$) oder nieder-Alkyliden ($C_2$-$C_4$) ist, wenn eine 5(6)-Doppelbindung anwesend ist, n 2 und $R^2$, $R^3$ und $R^4$ Methyl sind,

und von pharmazeutisch annehmbaren Salzen dieser Steroide, dadurch gekennzeichnet, dass man

a) eine Verbindung der Formel

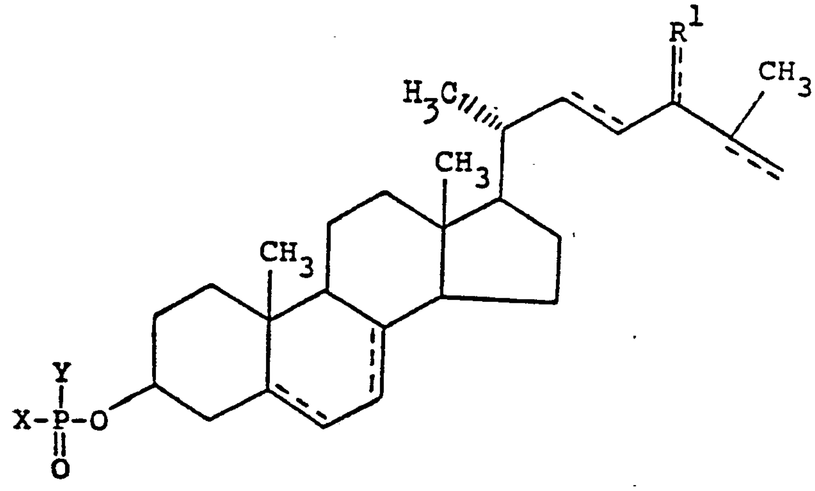

II

mit einem Salze der Formel

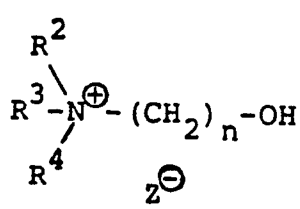

III

worin n, $R^1$, $R^2$, $R^3$, $R^4$ und die gestrichelten C-C-Bindungen die oben angegebenen Bedeutungen haben; X und Y Chlor, Brom oder Jod, oder X zusammen mit Y einen 1,2-Dimethyläthylendioxyrest, und Z nieder-Alkylsulfonyloxy, Arylsulfonyloxy, Perchloryloxy, Chlor, Brom oder Jod sind,

in Gegenwart einer Base umsetzt und das Produkt hydrolysiert, oder

b) eine Verbindung der Formel II, worin X eine metallierte Hydroxygruppe und Y eine Gruppe der Formel Hal-

$(CH_2)_n$-O- ist, worin Hal Chlor, Brom oder Jod ist; und n die obige Bedeutung hat, oder worin X zusammen mit Y einen Äthylendioxyrest bildet, mit einem Amin der Formel N($R^2$, $R^3$, $R^4$), worin $R^2$, $R^3$ und $R^4$ die obige Bedeutung haben, umsetzt, oder

c) in einer Verbindung der Formel II, worin X eine metallierte Hydroxygruppe und Y ein Rest der Formel

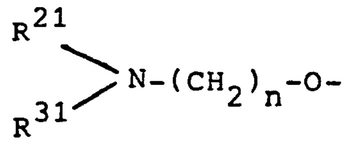

(Y)

ist,

worin eines von $R^{21}$ und $R^{31}$ eine Aminoschutzgruppe ist und das andere die Bedeutung von $R^2$ hat, oder $R^{21}$ und $R^{31}$ zusammen mit dem N-Atom eine geschützte Aminogruppe sind, und n und $R^2$ die obige Bedeutung haben,

die Aminoschutzgruppe abspaltet, und

d) ein erhaltenes Steroid der Formel I in dieser Form oder in Form eines Salzes isoliert.

2. Verfahren nach Anspruch 1 zur Herstellung von Steroiden der Formel I, worin $R^1$ Wasserstoff oder nieder-Alkyl ($C_1$-$C_4$), insbesondere Äthyl, ist, dadurch gekennzeichnet, dass man entsprechend substituierte Ausgangsmaterialien einsetzt.

3. Verfahren nach Anspruch 1 oder 2 zur Herstellung von Steroiden der Formel I, worin $R^2$, $R^3$ und $R^4$ nieder-Alkyl ($C_1$-$C_4$), insbesondere Methyl, sind, dadurch gekennzeichnet, dass man entsprechend substituierte Ausgangsmaterialien einsetzt.

4. Verfahren nach Anspruch 1, 2 oder 3 zur Herstellung von Steroiden der Formel I, worin n die Zahl 2 ist, dadurch gekennzeichnet, dass man entsprechend substituierte Ausgangsmaterialien einsetzt.

5. Verfahren nach einem der Ansprüche 1 - 4, zur Herstellung von gesättigten Steroiden der Formel I, dadurch gekennzeichnet, dass man ein entsprechendes Steroid der Formel II einsetzt.

6. Verfahren nach einem der Ansprüche 1 - 4, zur Herstellung eines 5(6)- und 22(23)-ungesättigten Steroids der Formel I, dadurch gekennzeichnet, dass man ein entsprechendes Steroid der Formel II einsetzt.

7. Verfahren nach einem der Ansprüche 1 - 5 zur Herstellung des 3ß-Stigmastanyloxy-phosphorylcholins, dadurch gekennzeichnet, dass man entsprechend substituierte Ausgangsmaterialien einsetzt.

8. Verfahren nach einem der Ansprüche 1 - 4 und 6 zur Herstellung des Stigmasta-5,22-dien-3β-yloxy-phosphorylcholins, dadurch gekennzeichnet, dass man entsprechend substituierte Ausgangsmaterialien einsetzt.

**Claims** for the Contracting States BE, CH, DE, FR, GB, IT, LI, LU, NL, SE

1. Steroids of the formula

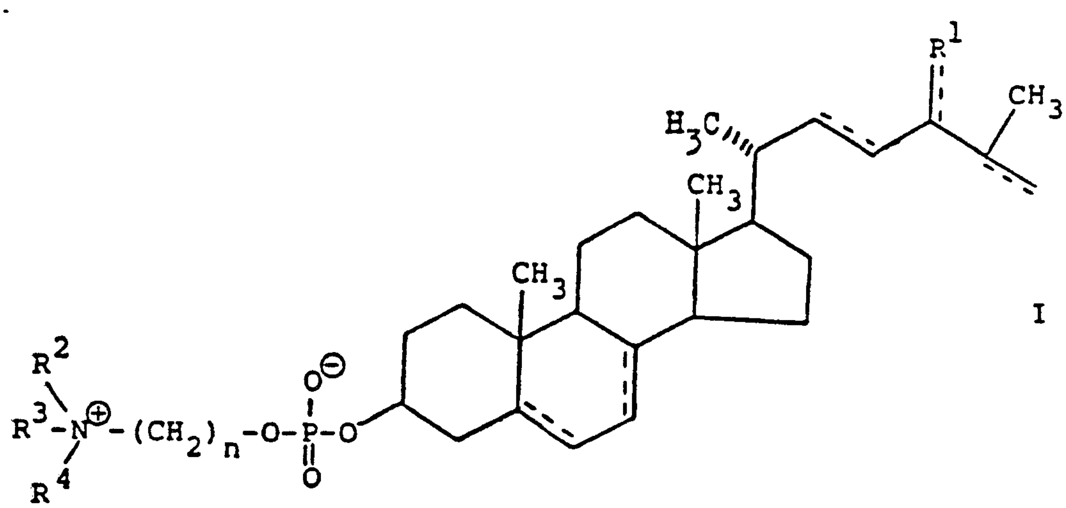

I

wherein n represents the number 2, 3 or 4: $R^1$ represents hydrogen, lower-alkyl ($C_1$-$C_4$) or lower-alkylidene ($C_2$-$C_4$); $R^2$, $R^3$ and $R^4$ represents hydrogen or lower-alkyl ($C_1$-$C_4$) and the dotted C-C bonds in the 5(6)-, 7(8)-, 22(23)-, 24(28)- and 25(26)-position are optional, whereby the B-ring can contain only one double bond and the side-chain is either saturated or is mono-unsaturated or is di-unsaturated in the 22(23), 25(26)-position; and

whereby $R^1$ is lower-alkyl ($C_1$-$C_4$) or lower-alkylidene ($C_2$-$C_4$) when a 5(6)-double bond is present, n is 2 and $R^2$, $R^3$ and $R^4$ are methyl,

and pharmaceutically acceptable salts of these steroids.

2. Steroids according to claim 1, wherein $R^1$ is hydrogen or lower-alkyl ($C_1$-$C_4$), especially ethyl.

3. Steroids according to claim 1 or 2, wherein $R^2$, $R^3$ and $R^4$ are lower-alkyl ($C_1$-$C_4$), especially methyl.

4. Steroids according to claim 1, 2 or 3, wherein n is the number 2.

5. Saturated steroids according to any one of claims 1 - 4.

6. 5(6)- and 22(23)-unsaturated steroids according to any one of claims 1 - 4.

7. 3β-Stigmastanyloxy-phosphorylcholine.

8. Stigmasta-5,22-dien-3β-yloxy-phosphorylcholine.

9. Products according to any one of claims 1 - 8 as pharmaceutically active substances.

10. Products according to any one of claims 1 - 8 as active substances which inhibit the intestinal resorption of cholesterol.

11. A process for the manufacture of products according to any one of claims 1-8, characterized by

a) reacting a compound of the formula

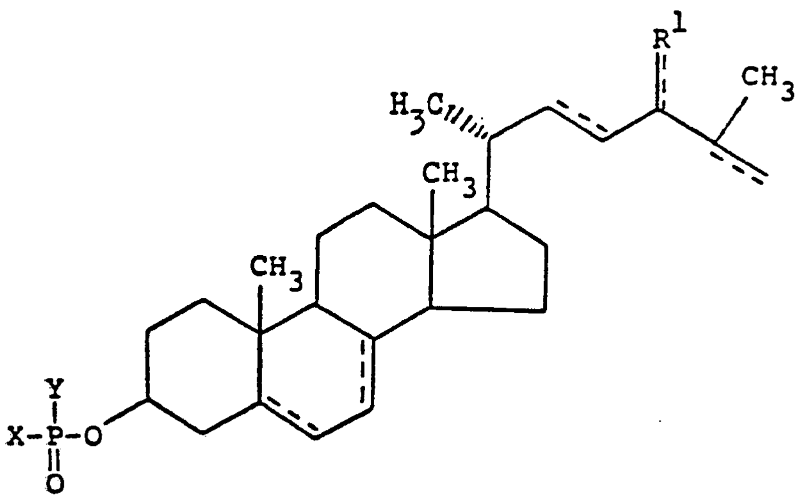

II

with a salt of the formula

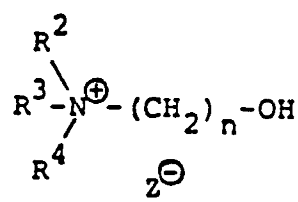

III

wherein n, $R^1$, $R^2$, $R^3$, $R^4$ and the dotted C-C bonds have the significances given in claim 1; X and Y are chlorine, bromine or iodine or X and Y together are a 1,2-dimethylethylenedioxy residue and Z is lower-alkylsulphonyloxy, arylsulphonyloxy, perchloryloxy, chlorine, bromine or iodine,

in the presence of a base and hydrolyzing the product, or

b) reacting a compound of formula II in which X is a metallized hydroxy group and Y is a group of the formula Hal-$(CH_2)_n$-O-, wherein Hal is chlorine, bromine or iodine; and n has the above significance, or in which X and Y together form an ethylenedioxy residue with an amine of the formula N($R^2$, $R^3$, $R^4$), wherein $R^2$, $R^3$ and $R^4$ have the above significance, or

c) cleaving off the amino protecting group in a compound of formula II in which X is a metallized hydroxy group and Y is a residue of the formula

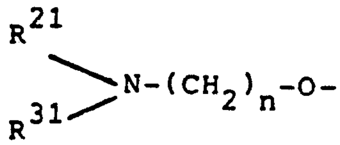

(Y)

wherein one of $R^{21}$ and $R^{31}$ is an amino protecting group and the other has the significance of $R^2$ or $R^{21}$ and $R^{31}$ together with the N-atom are a protected amino group and n and $R^2$ have the above significance,

and

d) isolating a steroid of formula I obtained in this form or in the form of a salt.

12. A medicament containing a compound according to any one of claims 1 - 8.

13. A medicament which inhibits the intestinal resorption of cholesterol, containing a compound according to any one of claims 1 - 8.

**Claims** for the Contracting State: AT

1. A process for the manufacture of steroids of the formula

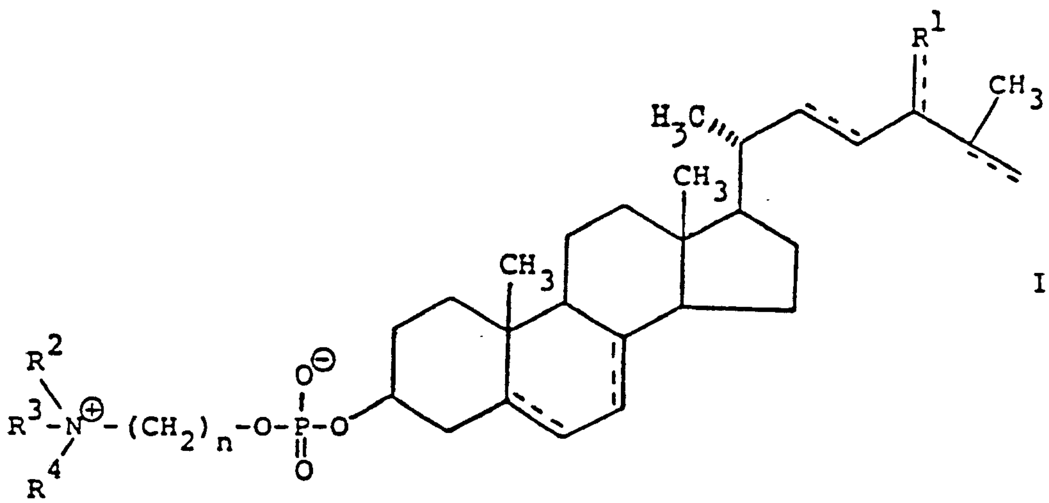

I

wherein n represents the number 2, 3 or 4; $R^1$ represents hydrogen, lower-alkyl ($C_1$-$C_4$) or lower-alkylidene ($C_2$-$C_4$); $R^2$, $R^3$ and $R^4$ represents hydrogen or lower-alkyl ($C_1$-$C_4$) and the dotted C-C bonds in the 5(6)-, 7(8)-, 22(23)-, 24(28)- and 25(26)-position are optional, whereby the B-ring can contain only one double bond and the side-chain is either saturated or is mono-unsaturated or is di-unsaturated in the 22(23), 25(26)-position; and whereby $R^1$ is lower-alkyl ($C_1$-$C_4$) or lower-alkylidene ($C_1$-$C_4$) when a 5(6)-double bond is present, n is 2 and $R^2$, $R^3$ and $R^4$ are methyl,

and of pharmaceutically acceptable salts of these steroids, characterized by

a) reacting a compound of the formula

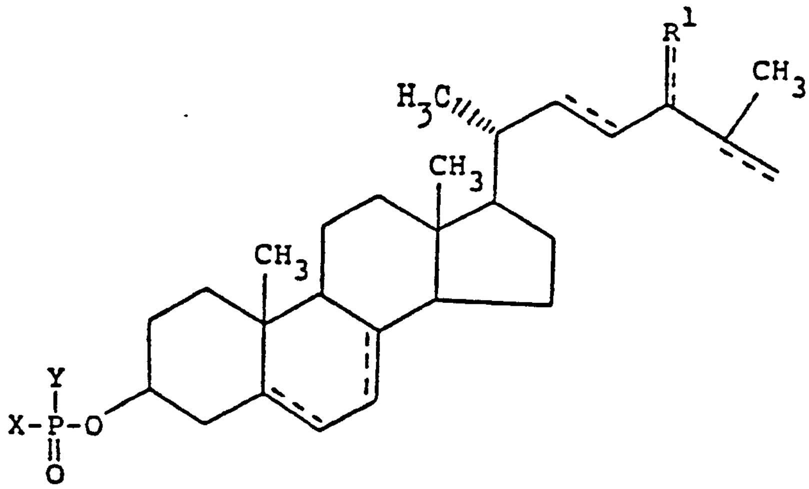

II

with a salt of the formula

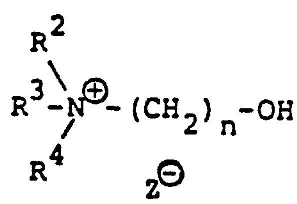

III

wherein n, $R^1$, $R^2$, $R^3$, $R^4$ and the dotted C-C bonds have the significances given above; X and Y are chlorine, bromine or iodine or X and Y together are a 1,2-dimethylethylenedioxy residue and Z is lower-alkylsulphonyloxy, arylsulphonyloxy, perchloryloxy, chlorine, bromine or iodine,

in the presence of a base and hydrolyzing the product, or

b) reacting a compound of formula II in which X is a metallized hydroxy group and Y is a group of the formula Hal-$(CH_2)_n$-O-, wherein Hal is chlorine, bromine or iodine; and n has the above significance, or in which X and Y together form an ethylenedioxy residue with an amine of the formula N($R^2$, $R^3$, $R^4$), wherein $R^2$, $R^3$ and $R^4$ have the above significance, or

c) cleaving off the amino protecting group in a compound of formula II in which X is a metallized hydroxy group and Y is a residue of the formula

wherein one of $R^{21}$ and $R^{31}$ is an amino protecting group and the other has the significance of $R^2$ or $R^{21}$ and $R^{31}$ together with the N-atom are a protected amino group and n and $R^2$ have the above significance,

and

d) isolating a steroid of formula I obtained in this form or in the form of a salt.

2. A process according to claim 1 for the manufacture of steroids of formula I in which $R^1$ is hydrogen or lower-alkyl ($C_1$-$C_4$), especially ethyl, characterized in that correspondingly substituted starting materials are used.

3. A process according to claim 1 or 2 for the manufacture of steroids of formula I in which $R^1$, $R^3$ and $R^4$ are lower-alkyl ($C_1$-$C_4$), especially methyl, characterized in that correspondingly substituted starting materials are used.

4. A process according to claim 1, 2 or 3 for the manufacture of steroids of formula I in which n is the number 2, characterized in that correspondingly substituted starting materials are used.

5. A process according to any one of claims 1 - 4 for the manufacture of saturated steroids of formula I, characterized in that a corresponding steroid of formula II is used.

6. A process according to any one of claims 1 - 4 for the manufacture of a 5(6)- and 22(23)-unsaturated steroid of formula I, characterized in that a corresponding steroid of formula II is used.

7. A process according to any one of claims 1 - 5 for the manufacture of 3β-stigmastanyloxy-phosphorylcholine, characterized in that correspondingly substituted starting materials are used.

8. A process according to any one of claims 1 - 4 and 6 for the manufacture of stigmasta-5,22-dien-3β-yloxy-phosphorylcholine, characterized in that correspondingly substituted starting materials are used.

**Revendications** pour les Etats Contractants: BE, CH, DE, FR, GB, IT, LI, LU, NL, SE

1. Stéroïdes de formule

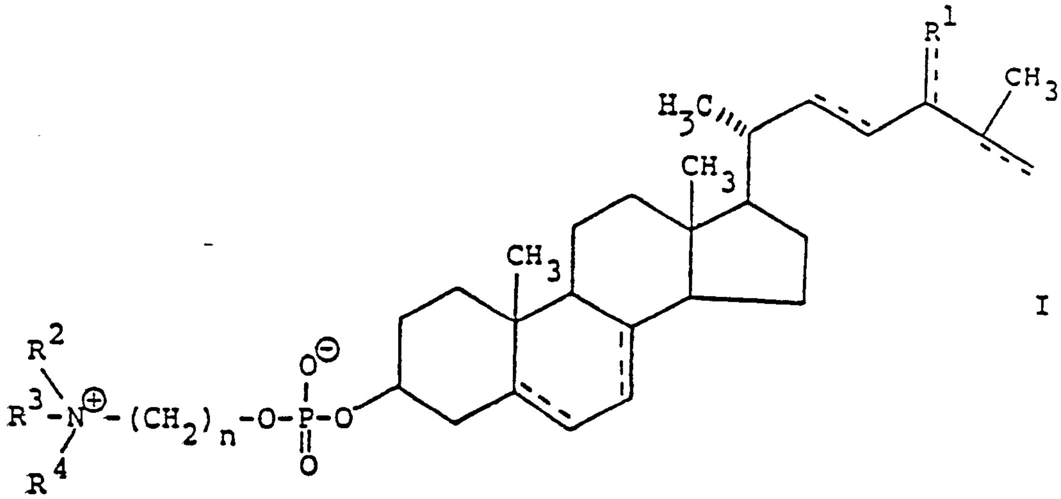

I

où n représente le nombre 2, 3 ou 4; $R^1$ représente un hydrogène, alcoyle inférieur en $C_1$ à $C_4$ ou alcoylidène inférieur en $C_2$ à $C_4$; $R^2$, $R^3$ et $R^4$ représentent un hydrogène ou un alcoyle inférieur en $C_1$ à $C_4$; et les liaisons C-C en pointillé sont facultatives en positions 5(6), 7(8), 22(23), 24(28) et 25(26), où le noyau B ne peut contenir qu'une double liaison et la chaîne latérale est saturée ou mono-insaturée, ou bi-insaturée en position 22(23), 25(26); et où $R^1$ est un alcoyle inférieur en $C_1$ à $C_4$ ou un alcoylidène inférieur en $C_2$ à $C_4$ lorsque une double liaison est présente en 5(6), n vaut 2 et $R^2$, $R^3$ et $R^4$ représentent un méthyle,

et les sels pharmaceutiquement acceptables de ces stéroïdes.

2. Stéroïdes selon la revendication 1, où $R^1$, un hydrogène ou un alcoyle inférieur en $C_1$ à $C_4$, en particulier un éthyle.

3. Stéroïdes selon la revendication 1 ou 2, où $R^2$, $R^3$ et $R^4$ représentent un alcoyle inferieur en $C_1$ à $C_4$, en particulier un méthyle.

4. Stéroïdes selon les revendications 1, 2 ou 3, où n est le nombre 2.

5. Stéroïdes saturés selon l'une des revendications 1 - 4.

6. Stéroïdes non saturés en 5(6) et 22(23) selon l'une des revendications 1 - 4.

7. 3β-stigmastanyloxy-phosphorylcholine.

8. Stigmasta-5,22-diène-3β-yloxy-phosphorylcholine.

9. Produits selon l'une des revendications 1 - 8 comme substances actives pharmaceutiques.

10. Produits selon l'une des revendications 1 - 8 comme substances actives inhibant la résorption intestinale du cholestérol.

11. Procédé de préparation de produits selon l'une des revendications 1 - 8, caractérisé en ce que

a) on fait réagir un composé de formule

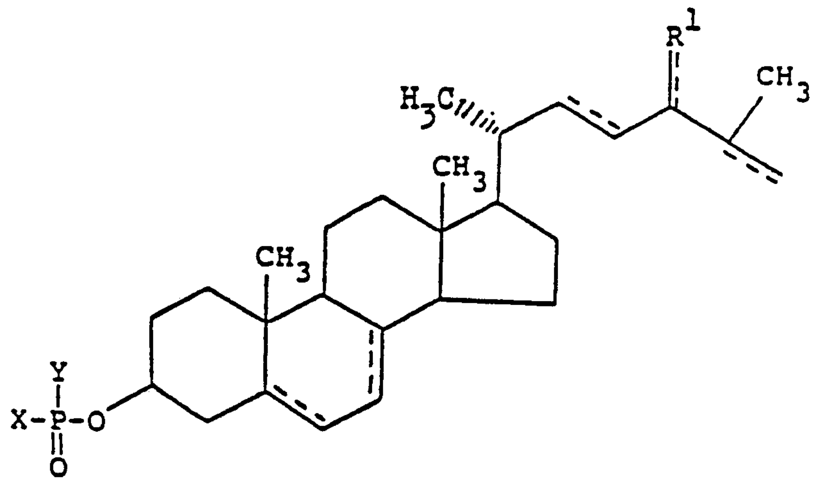

II

avec un sel de formule

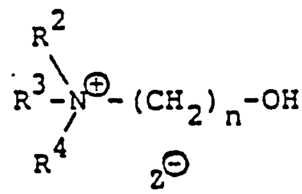

III

où n, $R^1$, $R^2$, $R^3$, $R^4$ et les liaisons C-C en pointillé ont les significations données dans la revendication 1; X et Y représentent un chlore, un brome ou un iode, ou X avec Y représentent ensemble un radical 1,2-diméthyléthylènedioxy, et Z est un alcoyle inférieur-sulfonyloxy, arylsulfonyloxy, perchloryloxy, chlore, brome ou iode, en présence d'une base et on hydrolyse le produit, ou

b) on fait réagir un composé de formule II, où X représente un groupe hydroxy métallé et Y un groupe de formule Hal-$(CH_2)_n$-O- , où Hal est un atome de chlore, de brome ou d'iode; et n a la signification donnée ci-dessus, ou bien où X forme avec Y un radical éthylènedioxy, avec une amine de formule N($R^2$, $R^3$, $R^4$), où $R^2$, $R^3$ et $R^4$ ont la signification donnée ci-dessus, ou

c) dans un composé de formule II, où X est un groupe hydroxy métallé et Y un radical de formule

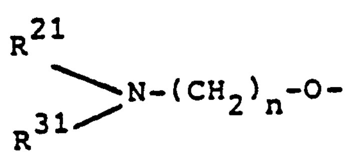

(Y)

où l'un des radicaux $R^{21}$ et $R^{31}$ est un groupe protecteur d'amino et l'autre a la signification de $R^2$, ou bien $R^{21}$ et $R^{31}$ représentent ensemble avec l'atome d'azote un groupe amino protégé, et n et $R^2$ ont la signification donnée ci-dessus,

on sépare le groupe protecteur d'amino, et

d) on isole un stéroïde de formule I obtenu sous cette forme ou sous la forme d'un sel.

12. Médicament contenant un composé selon l'une des revendications 1 - 8.

13. Agent inhibant la résorption intestinale du cholestérol, contenant un composé selon l'une des revendications 1 - 8.

**Revendications** pour l'Etat Contractant: AT

1. Procédé de préparation de stéroïdes de formule

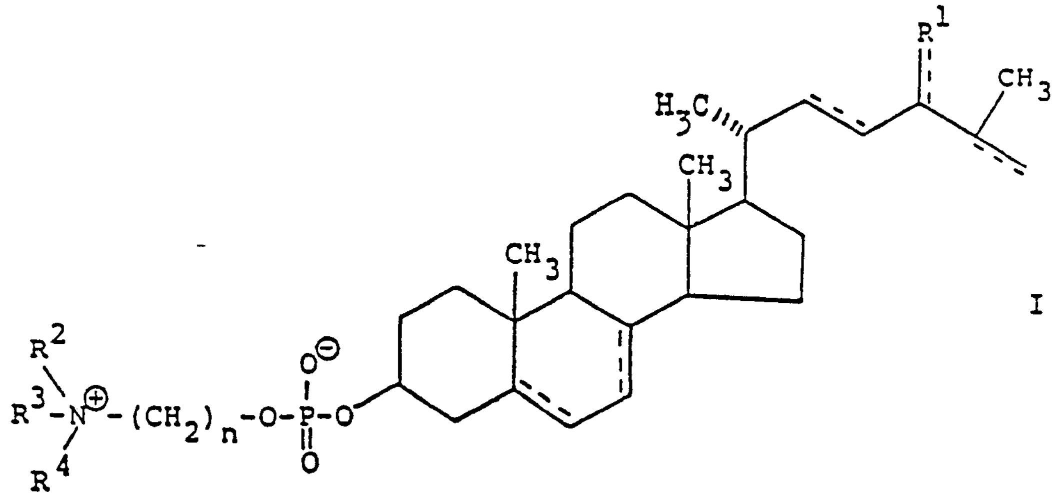

I

où n représente le nombre 2, 3 ou 4; $R^1$ représente un hydrogène, alcoyle inférieur en $C_1$ à $C_4$ ou alcoylidène inférieur en $C_2$ à $C_4$; $R^2$, $R^3$ et $R^4$ représentent un hydrogène ou un alcoyle inférieur en $C_1$ à $C_4$; et les liaisons C-C en pointillé sont facultatives en positions 5(6), 7(8), 22(23), 24(28) et 25(26), où le noyau B ne peut contenir qu'une double liaison et la chaîne latérale est saturée ou mono-insaturée, ou bi-insaturée en position 22(23), 25(26); et où $R^1$ est un alcoyle inférieur en $C_1$ à $C_4$ ou un alcoylidène inférieur en $C_2$ à $C_4$ lorsque une double liaison est présente en 5(6), n vaut 2 et $R^2$, $R^3$ et $R^4$ représentent un méthyle,

et des sels pharmaceutiquement acceptables de ces stéroïdes,

caractérisé en ce que

a) on fait réagir un composé de formule

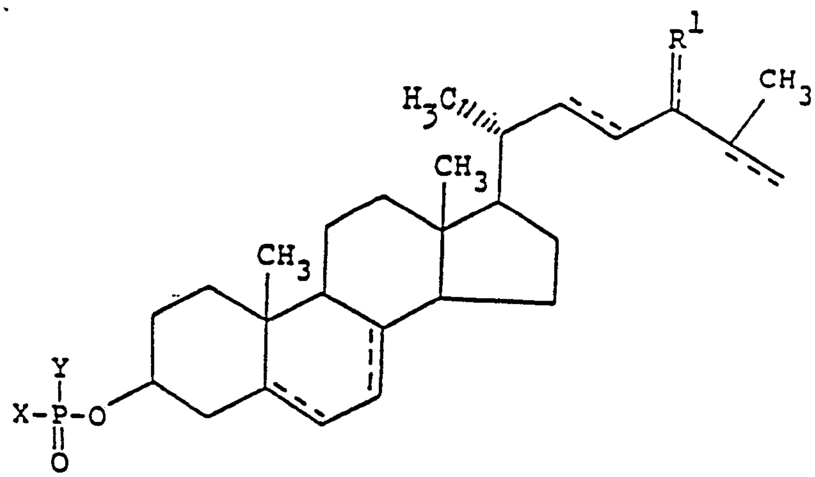

II

avec un sel de formule

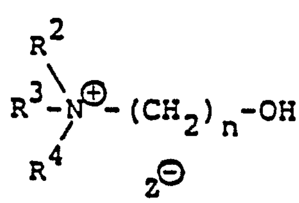

III

où n, $R^1$, $R^2$, $R^3$, $R^4$ et les liaisons C-C en pointillé ont les significations données dans la revendication 1; X et Y représentent un chlore, un brome ou un iode, ou X avec Y représentent ensemble un radical 1,2-diméthyléthylènedioxy, et Z est un alcoyle inférieur-sulfonyloxy, arylsulfonyloxy, perchloryloxy, chlore, brome ou iode, en présence d'une base et ou hydrolyse le produit, ou

b) on fait réagir un composé de formule II, où X représente un groupe hydroxy métallé et Y un groupe de

formule Hal-$(CH_2)_n$-O- , où Hal est un atome de chlore, de brome ou d'iode; et n a la signification donnée ci-dessus, ou bien où X forme avec Y un radical éthylènedioxy, avec une amine de formule N($R^2$, $R^3$, $R^4$), où $R^2$, $R^3$ et $R^4$ ont la signification donnée ci-dessus, ou

c) dans un composé de formule II, où X est un groupe hydroxy métallé et Y un radical de formule

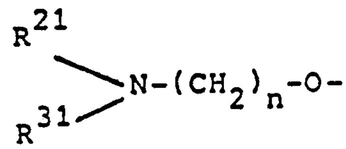

(Y)

où l'un des radicaux $R^{21}$ et $R^{31}$ est un groupe protecteur d'amino et l'autre a la signification de $R^2$, ou bien $R^{21}$ et $R^{31}$ représentent ensemble avec l'atome d'azote un groupe amino protégé, et n et $R^2$ ont la signification donnée ci-dessus,

on sépare le groupe protecteur d'amino, et

d) on isole un stéroïde de formule I obtenu sous cette forme ou sous la forme d'un sel.

2. Procédé selon la revendication 1 de préparation de stéroïdes de formule I, où $R^1$ est un hydrogène ou un alcoyle inférieur en $C_1$ à $C_4$, en particulier un éthyle, caractérisé en ce qu'on utilise des produits de départ substitués de façon correspondante.

3. Procédé selon la revendication 1 ou 2 pour la préparation de stéroïdes de formule I, où $R^2$, $R^3$ et $R^4$ représentent un alcoyle inférieur en $C_1$ à $C_4$, en particulier un méthyle, caractérisé en ce qu'on utilise des produits de départ substitués de façon correspondante.

4. Procédé selon les revendications 1, 2 ou 3 pour la préparation de stéroïdes de formule I, où n est le nombre 2, caractérisé en ce qu'on utilise des produits de départ substitués de façon correspondante.

5. Procédé selon l'une des revendications 1 - 4, pour la préparation de stéroïdes saturés de formule I, caractérisé en ce qu'on utilise un stéroïde correspondant de formule II.

6. Procédé selon l'une des revendications 1 - 4, pour la préparation d'un stéroïde de formule I non saturé en 5(6) et 22(23), caractérisé en ce qu'on utilise un stéroïde correspondant de formule II.

7. Procédé selon l'une des revendications 1 - 5 pour la préparation de la 3β-stigmastanyloxy-phosphorylcholine, caractérisé en ce qu'on utilise des produits de départ substitués de façon correspondante.

8. Procédé selon l'une des revendications 1 - 4 et 6 pour la préparation de la stigmasta-5,22-diène-3β-yloxy-phosphorylcholine, caractérisé en ce qu'on utilise des produits de départ substitués de façon correspondante.